# EUROPÄISCHE PATENTSCHRIFT

(11) **EP 0 822 245 B1**
(45) Veröffentlichungstag und Bekanntmachung des Hinweises auf die Patenterteilung: **14.11.2001**
(21) Anmeldenummer: 97108420.7
(22) Anmeldetag: 24.05.1997
(51) Int. Cl.: C10G 7/08, C07C 7/08, C07C 15/00

(54) **Verfahren zur Gewinnung reiner Kohlenwasserstoffe aus einem aromaten- und nichtaromatenhaltigen Kohlenwasserstoffgemisch**
Process for obtaining pure aromatics from hydrocarbon mixtures containing aromatics and non-aromatics
Procédé d'obtention d'aromates pures à partir de mélanges d'aromates et de non-aromates

(30) Priorität: 31.07.1996 DE 19630771
(43) Veröffentlichungstag der Anmeldung: 04.02.1998
(73) Patentinhaber: Krupp Uhde GmbH, 44141 Dortmund (DE)
(72) Erfinder: Donnermeyer, Stefan, 45144 Essen (DE); Ludolph, Jürgen, 45149 Essen (DE); Vollmer, Hans-Jürgen Dr., 45133 Essen (DE)
(74) Vertreter: Albrecht, Rainer Harald, Dr.-Ing.

(56) Entgegenhaltungen:
- EP-A- 0 491 196
- DE-A- 1 543 104
- US-A- 3 259 555
- US-A- 4 776 927

## Beschreibung

Die Erfindung betrifft ein Verfahren zur Gewinnung reiner Kohlenwasserstoffe aus einem aromaten- und nichtaromatenhaltigen Kohlenwasserstoffgemisch durch Extraktivdestillation nach dem Oberbegriff des Patentanspruches 1.

Die Extraktivdestillation verschiedener aromaten- und nichtaromatenhaltiger Kohlenwasserstoffgemische mit N-substituierten Morpholinen als selektives Lösungsmittel wird in großem Umfang in großtechnischen Anlagen zur Gewinnung sehr reiner Aromaten eingesetzt. Als Kohlenwasserstoffgemische werden insbesondere Reformatbenzin, Pyrolysebenzin oder Kokereirohbenzol eingesetzt. Diese Kohlenwasserstoffgemische enthalten neben verschiedenen Aromaten auch nichtaromatische Bestandteile, die insbesondere aus Paraffinen, Cyclopraffinen, Olefinen, Diolefinen und organischen Schwefelverbindungen bestehen. Mit der Extraktivdestillation werden die Kohlenwasserstoffgemische in Aromaten einerseits und Nichtaromaten andererseits aufgetrennt. Insofern eignet sich diese Extraktivdestillation sowohl zur Gewinnung von reinen Aromaten als auch zur Gewinnung von reinen Nichtaromaten, wie z.B. von Olefinen und Diolefinen. Bei der Extraktivdestillation reichern sich die Aromaten aus dem eingesetzten Kohlenwasserstoffgemisch mit der Hauptmenge des selektiven Lösungsmittels im Sumpf der Extraktivdestillationskolonne als Extrakt an. Die Nichtaromaten aus dem eingesetzten Kohlenwasserstoffgemisch reichern sich dagegen im Kopf der Extraktivdestillationskolonne als Raffinat an. - In der Praxis ist es bekannt, bei Extraktivdestillationen das selektive Lösungsmittel am Kopf der Extraktivdestillationskolonne zuzuführen. Dies führt jedoch dazu, dass das am Kopf der Extraktivdestillationskolonne anfallende Raffinat noch nicht zu vernachlässigende Lösungsmittelreste enthält. Dieser Lösungsmittelanteil im Raffinat wird bei diesen aus der Praxis bekannten Extraktivdestillationen weitgehend zurückgewonnen, was aufwendig ist.

Bei dem bekannten Verfahren, von dem die Erfindung ausgeht (DE 40 40 145 A1), soll gegenüber dem aus der Praxis bekannten Verfahren sowohl die Extraktivdestillation als auch die Entfernung der Lösungsmittelreste aus dem Raffinat in einer einzigen Kolonne durchgeführt werden. Hierzu weist die Extraktivdestillationskolonne einen zusätzlichen oberen Kolonnenteil auf und wird das selektive Lösungsmittel über eine Lösungsmittelzuführung unterhalb dieses oberen Kolonnenteiles in die Extraktivdestillationskolonne eingeführt. Dieses eingeführte Lösungsmittel fließt über die Böden der Kolonne nach unten, wobei es die Aromaten zum Sumpf der Extraktivdestillationskolonne mitnimmt. Die nichtaromatischen Kohlenwasserstoffe aus dem eingesetzten Kohlenwasserstoffgemisch steigen dagegen in der Extraktivdestillationskolonne dampfförmig nach oben. Das obere Kolonnenteil oberhalb der Lösungsmittelzuführung dient dazu, dass aus diesen nicht aromatischen Kohlenwasserstoffen die Lösungsmittelreste entfernt werden können. Das aus DE 40 40 145 A1 bekannte Verfahren hat sich im Wesentlichen bewährt, ist jedoch verbesserungsfähig. Dieses bekannte Verfahren weist insbesondere dann Nachteile auf, wenn ein Kohlenwasserstoffgemisch mit hohem Aromatengehalt eingesetzt wird, also beispielsweise Kokereirohbenzol mit einem Aromatengehalt von etwa 85% oder bereits vorgereinigtes Benzol mit einem Aromatengehalt von mehr als 95%. Bei derartigen eingesetzten Kohlenwasserstoffgemischen ist der Anteil an Nichtaromaten im Verhältnis zur Menge des selektiven Lösungsmittels sehr gering. Bei der Extraktivdestillation werden die Aromaten von dem von oben aufgegebenen selektiven Lösungsmittel zum Sumpf der Extraktivdestillationskolonne mitgenommen. Die Nichtaromaten steigen, wie oben bereits dargelegt, dampfförmig zum Kopf der Extraktivdestillationskolonne auf. Auf den einzelnen Böden der Extraktivdestillationskolonne stellen sich einzelne Gleichgewichte ein, die insbesondere von der Temperatur und von den Konzentrationen der Substanzen abhängig sind. Finden bei der Extraktivdestillation nach dem bekannten Verfahren Unregelmäßigkeiten in der eingestellten Beheizung der Kolonne statt oder treten Veränderungen der vorgegebenen Lösungsmitteltemperatur auf oder ändert sich die Menge des zugeführten Lösungsmittels, so werden diese Gleichgewichte gestört. Diese Störungen der Gleichgewichte wirken sich insbesondere beachtlich auf die Extraktivdestillation aus, wenn ein Kohlenwasserstoffgemisch mit sehr hohem Aromatengehalt eingesetzt wird, wie beispielsweise vorgereinigtes Benzol mit einem Aromatengehalt von mehr als 95%. Aufgrund des hohen Aromatengehaltes und des niedrigen Nichtaromatengehaltes, insbesondere in Bezug auf die Menge an selektivem Lösungsmittel, führen diese Schwankungen bzw. Störungen dazu, dass die in hoher Konzentration vorliegenden Aromaten als Folge der gestörten Gleichgewichte nicht mehr vollständig von dem selektiven Lösungsmittel in den Sumpf mitgenommen werden können. Vielmehr kann insbesondere leicht siedendes Benzol, das im Vergleich zu den Nichtaromaten eine geringere Siedetemperatur aufweist, in den oberen Kolonnenteil über der Lösungsmittelzuführung eintreten. Dadurch gelangt insbesondere Benzol in den Kopf der Extraktivdestillationskolonne, so dass im Kopf nunmehr ein Gemisch aus Aromaten und Nichtaromaten vorliegt. Schon eine geringe Menge von Aromaten, die aufgrund obengenannter Schwankungen in den oberen Kolonnenteil (oberhalb der ersten Lösungsmittelaufgabe) gelangen, führen bei den relativ geringen Mengen an Nichtaromaten, die hier vorausgesetzt werden, zu relativ hohen Aromatengehalten im Raffinanat. Zusätzlich bedeuten sie auch eine Einbuße der Aromatenausbeute.

Es ist ferner bekannt, das selektive Lösungsmittel in Teilströmen der Extraktivdestillationskolonne zuzuführen. Bei einem aus US 4 776 927 bekannten Verfahren zur Extraktivdestillation werden 60 bis 90% des insgesamt zugeführten selektiven Lösungsmittels am obersten Boden der Kolonne aufgegeben. 10 bis 40% der Lösungsmittelmenge wird in Teilströme aufgeteilt, die einem unterseitig anschließenden Kolonnenabschnitt zugeführt werden. Bei einem in DE-A 15 43 104 beschriebenen Verfahren zur Extraktivdestillation wird das selektive Lösungsmittel in Form eines mengenmäßig größeren Teilstromes einem Kolonnenabschnitt unterhalb des Zulaufes des zu trennenden Kohlenwasserstoffgemisches zugeführt. Der restliche Teil des Lösungsmittels, nämlich 10 bis 40%, bezogen auf die gesamte Lösungsmittelmenge, werden auf Teilströme aufgeteilt, die einem oberen Kolonnenabschnitt zugeführt werden. Sofern der Aromatengehalt des am Kopf der Kolonne abgezogenen Raffinatstromes von einem Vorgabewert abweicht, wird die Temperatur der Lösungsmittelteilströme geregelt.

Der Erfindung liegt das technische Problem zugrunde, ein Verfahren zur Extraktivdestillation anzugeben, mit dem trotz Unregelmäßigkeiten und Schwankungen der Extraktivdestillationsbedingungen, insbesondere Temperatur- und Konzentrationsschwankungen, eine saubere Trennung von Aromaten und Nichtaromaten und eine entsprechend hohe Ausbeute an Aromaten und Nichtaromaten auf relativ einfache und wenig aufwendige Weise erreicht wird.

Gegenstand der Erfindung und Lösung dieser Aufgabe ist ein Verfahren nach Anspruch 1. Bei dem erfindungsgemäßen Verfahren werden aromatenreiche Kohlenwasserstoffgemische mit einem mit einem Aromatengehalt von über 85% eingesetzt. Vorzugsweise wird vorgereinigtes Benzol mit einem Aromatengehalt von mehr als 95% und/oder Kokereirohbenzol mit einem Aromatengehalte von etwa 85% eingesetzt. In der Regel weisen die Kohlenwasserstoffgemische insbesondere Aromaten mit 6 bis 8 Kohlenstoffatomen auf.

Vorzugsweise werden N-substituierte Morpholine mit nicht mehr als 7 Kohlenstoffatomen im Substituenten als selektives Lösungsmittel verwendet. Nach bevorzugter Ausführungsform der Erfindung wird N-Formylmorpholin als selektives Lösungsmittel eingesetzt.

Die Erfindung geht von der Erkenntnis aus, dass bei dem aus DE 40 40 145 A1 bekannten Verfahren in dem Kolonnenteil über der Lösungsmittelzuführung kein zugeführtes selektives Lösungsmittel vorhanden ist und daher in diesem Kolonnenteil im Wesentlichen Destillationsbedingungen und keine Extraktivdestillationsbedingungen vorliegen. Aus diesem Grunde kann beispielsweise leicht siedendes Benzol bei den oben beschriebenen Schwankungen der Extraktivdestillationsbedingungen in den Kopf der Kolonne gelangen und wird daher in nachteilhafter Weise das Benzol mit den Nichtaromaten im Kopf vermischt. Die Erfindung beruht fernerhin auf der Erkenntnis, dass durch Zuführung eines zweiten Teilstromes des selektiven Lösungsmittels über eine zweite Lösungsmittelzuführung, die oberhalb der ersten Lösungsmittelzuführung angeordnet ist, im Bereich zwischen den beiden Lösungsmittelzuführungen Extraktivdestillationsbedingungen verwirklicht werden können. Durch diesen zusätzlichen Abschnitt der Kolonne mit Extraktivdestillationsbedingungen kann erreicht werden, dass Aromaten und insbesondere Benzol nicht mehr in den Kopf der Kolonne gelangen können. Die Aromaten werden in den Kolonnenunterteil zurückgewaschen. Auch bei größeren Schwankungen der Temperaturen und Konzentrationen in der Extraktivdestillationskolonne wird dieses Ergebnis durch den zusätzlichen Extraktivdestillationsbereich zwischen den beiden Lösungsmittelzuführungen erzielt. Hierfür reicht es überraschenderweise aus, dass über die zweite Lösungsmittelzuführung nur eine verhältnismäßig geringe Menge des selektiven Lösungsmittels zugeführt werden muss.

Den Fachmann muss es überraschen, dass durch Zuführung relativ geringer Mengen an selektivem Lösungsmittel über die zweite Lösungsmittelzuführung die Aromaten effektiv aus dem Kopf der Kolonne ferngehalten werden können. Aufgrund des zusätzlichen Lösungsmittelteilstroms haben Schwankungen bezüglich der Temperatur und der zugeführten Lösungsmittelmenge kaum Auswirkungen auf den Aromatengehalt im Kopf der Kolonne. Die verhältnismäßig geringe Lösungsmittelmenge führt auch dazu, dass sich der zweite Teilstrom genauer einstellen und regeln lässt. Zwischen Kolonnenoberteil und Kolonnenunterteil der Extraktivdestillationskolonne kann, z.B. über einen Kocher, Wärme zugeführt werden.

Im Ergebnis wird mit dem erfindungsgemäßen Verfahren eine saubere Trennung von Aromaten, die im Sumpf der Kolonne anfallen, und Nichtaromaten, die im Kopf der Kolonne anfallen, erzielt. Dementsprechend wird eine hohe Ausbeute sowohl an reinen Aromaten als auch an reinen Nichtaromaten erreicht. Hervorzuheben ist, dass diese Vorteile auf einfache und wenig aufwendige Weise verwirklicht werden können. Gegenüber der im Rahmen des Verfahrens nach DE 40 40 145 A1 eingesetzten Extraktivdestillationskolonne ist die erfindungsgemäß eingesetzte Extraktivdestillationskolonne lediglich um etwa 5 bis 10 theoretische Böden bzw. Trennstufen erhöht. Abgesehen von der zweiten Lösungsmittelzuführung ist ein weiterer apparativer Aufwand im Hinblick auf die Anlage nicht erforderlich. Gegenüber dem bekannten Verfahren ist auch ein zusätzlicher Einsatz von selektiven Lösungsmittelmengen nicht erforderlich, da nach dem erfindungsgemäßen Verfahren die eingesetzte Lösungsmittelmenge lediglich in zwei Teilströme aufgeteilt wird, die über die beiden Lösungsmittelzuführungen in die Extraktivdestillationskolonne eingeführt werden.

Es versteht sich im übrigen, dass im Rahmen des erfindungsgemäßen Verfahrens zwischen der zweiten Lösungsmittelzuführung und dem Kopf der Extraktivdestillationskolonne ein Abschnitt des Kolonnenoberteils eingerichtet ist, in dem Destillationsbedingungen herrschen und der dem Zweck dient, dass selektives Lösungsmittel von den Nichtaromaten destillativ abgetrennt wird. - Im einzelnen bestehen mehrere Möglichkeiten zur Durchführung des erfindungsgemäßen Verfahrens. Es liegt im Rahmen der Erfindung, dass das aus dem Sumpf der Extraktivdestillationskolonne abgezogene Extrakt aus Aromaten und selektivem Lösungsmittel einer Abtriebskolonne zugeführt wird und dass in der Abtriebskolonne die Aromaten von dem selektiven Lösungsmittel getrennt werden. Zweckmäßigerweise wird die Abtriebskolonne unter Vakuum betrieben und fällt dabei das Aromatenprodukt im Kopf der Abtriebskolonne an. Das selektive Lösungsmittel wird dabei in den Sumpf der Abtriebskolonne überführt. Nach bevorzugter Ausführungsform der Erfindung wird das aus dem Sumpf der Extraktivdestillationskolonne abgezogene selektive Lösungsmittel im Kreislauf geführt und über die Lösungsmittelzuführungen wieder in die Extraktivdestillationskolonne eingeführt. Dazu wird zweckmäßigerweise das im Sumpf der Abtriebskolonne anfallende selektive Lösungsmittel über die erste und die zweite Lösungsmittelzuführung wieder in die Extraktivdestillationskolonne eingeführt. Zweckmäßigerweise wird dabei das aus der Abtriebskolonne abgezogene Lösungsmittel über einen geeigneten Wärmetauscher geführt, um das selektive Lösungsmittel abzukühlen und die gewünschte Temperatur des Lösungsmittels einzustellen.

Es liegt auch im Rahmen der Erfindung, dass das eingesetzte Kohlenwasserstoffgemisch vor Einleitung in die Extraktivdestillationskolonne in einem Trennbehälter entspannt und dadurch in eine flüssige umd dampfförmige Phase zerlegt werden kann. Die beiden Phasen können anschließend getrennt voneinander in die Extraktivdestillationskolonne eingeleitet werden. Dabei wird die dampfförmige Phase unterhalb der Zuführung für die flüssige Phase in die Extraktivdestillationskolonne eingeleitet.

Es versteht sich, dass bei dem erfindungsgemäßen Verfahren die Temperatur des zugeführten selektiven Lösungsmittels sowie die Temperatur des eingeführten Kohlenwasserstoffgemisches durch entsprechende Wärmetauscher eingestellt werden können. Die Beheizung der Extraktivdestillationskolonne erfolgt auf übliche Weise.

Im folgenden wird die Erfindung anhand einer lediglich ein Ausführungsbeispiel darstellenden Zeichnung ausführlicher erläutert. Es zeigen in schematischer Darstellung
- Fig. 1: eine Vorrichtung zur Durchführung des Verfahrens zur Gewinnung reiner Kohlenwasserstoffe nach dem Stand der Technik,
- Fig. 2: eine Vorrichtung zur Durchführung des erfindungsgemäßen Verfahrens zur Gewinnung reiner Kohlenwasserstoffe.

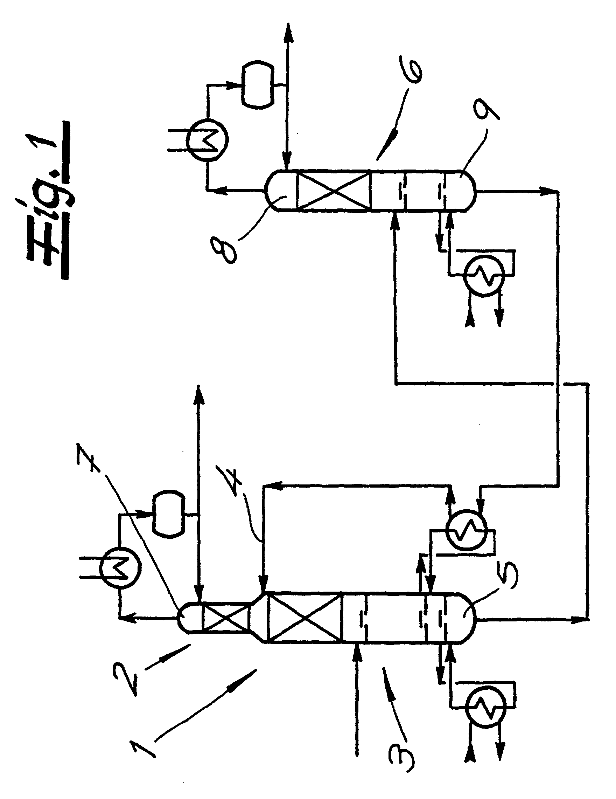

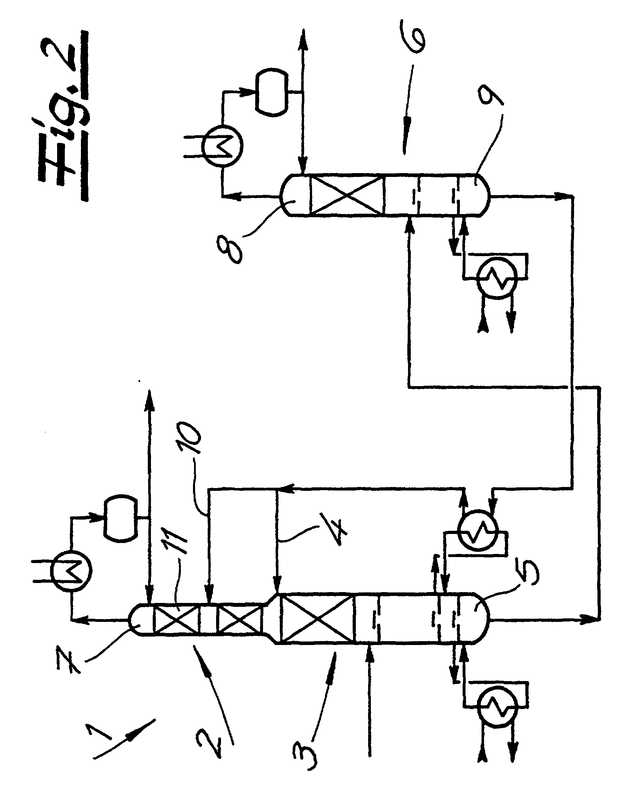

Die Fig. 1 und 2 zeigen eine Vorrichtung zur Durchführung von Verfahren zur Gewinnung reiner Kohlenwasserstoffe aus einem aromaten- und nichtaromatenhaltigen Kohlenwasserstoffgemisch, insbesondere aus einem aromatenreichen Kohlenwasserstoffgemisch. Im Rahmen der Verfahren findet eine Extraktivdestillation in einer Extraktivdestillationskolonne 1 statt, bei der zwischen einem Kolonnenoberteil 2 und einem Kolonnenunterteil 3 eine Lösungsmittelzuführung 4 angeordnet ist, über die das selektive Lösungsmittel in die Extraktivdestillationskolonne 1 eingeführt wird. Das Kolonnenoberteil 2 kann als separate Kolonne ausgeführt sein. Als selektives Lösungsmittel wird vorzugsweise N-Formylmorpholin eingesetzt. Durch die Zugabe des selektiven Lösungsmittels in die Extraktivdestillationskolonne 1 werden die Siedepunkte von Aromaten und Nichtaromaten in dem eingesezten Kohlenwasserstoffgemisch so gegeneinander verschoben, dass sie sich in der Extraktivdestillationskolonne trennen lassen. Die Aromaten werden von dem selektiven Lösungsmittel in den Sumpf 5 der Extraktivdestillationskolonne 1 geführt. Das im Sumpf 5 der Extraktivdestillationskolonne 1 anfallene Extrakt aus Aromaten und selektivem Lösungsmittel wird abgezogen und einer Abtriebskolonne 6 zugeführt. Die Nichtaromaten steigen in der Extraktivdestillationskolonne 1 dampfförmig nach oben und fallen als Raffinat im Kopf 7 der Extraktivdestillationskolonne 1 an und können von dort abgezogen werden. In der Abtriebskolonne 6 wird das aus dem Sumpf 5 abgezogene Extrakt aus Aromaten und selektivem Lösungsmittel vorzugsweise im Vakuum in die Aromaten und das selektive Lösungsmittel aufgetrennt. Die Aromaten fallen dabei im Kopf 8 der Abtriebskolonne 6 an und können von dort abgezogen werden. Das selektive Lösungsmittel fällt dagegen im Sumpf 9 der Abtriebskolonne 6 an. Aus dem Sumpf 9 wird das selektive Lösungsmittel abgezogen und über die Lösungsmittelzuführung 4, 10 wieder in die Extraktivdestillationskolonne 1 eingeleitet. Das selektive Lösungsmittel wird also im Kreislauf geführt. Es versteht sich, dass geeignete Wärmetauscher vorgesehen sind, um das selektive Lösungsmittel vor Wiedereinführung in die Extraktivdestillationskolonne 1 auf eine vorgegebene Temperatur abzukühlen. Sowohl in der Fig. 1 als auch in der Fig. 2 ist das über der Lösungsmittelzuführung 4 angeordnete Kolonnenoberteil 2 als Kolonnenaufsatz ausgeführt, der häufig einen geringeren Durchmesser als das Kolonnenunterteil 3 der Extraktivdestillationskolonne 1 aufweist.

In der Fig. 1 ist die Vorrichtung zur Durchführung des Verfahrens nach DE 40 40 145 A1 dargestellt, von dem die Erfindung ausgeht. Hier ist lediglich eine einzige Lösungsmittelzuführung 4 zwischen Kolonnenoberteil 2 und Kolonnenunterteil 3 vorgesehen. In dem Kolonnenoberteil 2 über der Lösungsmittelzuführung 4 herrschen im wesentlichen Destillationsbedingungen und dieses Kolonnenoberteil dient zur Entfernung des selektiven Lösungsmittels aus den Nichtaromaten, die sich im Kopf 7 der Kolonne ansammeln. Wie bereits oben erläutert, zeichnet sich dieses Verfahren durch den Nachteil aus, dass bei Schwankungen der Extraktivdestillationsbedingungen, insbesondere Schwankungen der Beheizung der Extraktivdestillationskolonne 1, der Lösungsmitteltemperatur und/oder der zugeführten Menge an selektivem Lösungsmittel, Aromaten in das Kolonnenoberteil 2 gelangen können und sich somit in unerwünschter Weise mit den Nichtaromaten im Kopf 7 vermischen. Dieser nachteilhafte Effekt tritt insbesondere auf, wenn bei dem bekannten Verfahren aromatenreiche Kohlenwasserstoffgemische eingesetzt werden.

In der Fig. 2 ist eine Vorrichtung zur Durchführung des erfindungsgemäßen Verfahrens dargestellt. Erfindungsgemäß wird in dem Kolonnenoberteil 2 oberhalb der ersten Lösungsmittelzuführung 4 ein zweiter Teilstrom des selektiven Lösungsmittels über eine zweite Lösungsmittelzuführung 10 in die Extraktivdestillationskolonne 1 eingeführt. Die mit dem zweiten Teilstrom zugeführte Lösungsmittelmenge beträgt vorzugsweise lediglich 0,5 bis 10% der gesamten über die Lösungsmittelzuführungen 4, 10 zugeführten Lösungsmittelmenge. Überraschenderweise kann durch diese relativ wenig aufwendige Maßnahme erreicht werden, dass auch bei den oben beschriebenen Schwankungen der Extraktivdestillationsbedingungen die Aromaten sehr vollständig in den Sumpf 5 der Extraktivdestillationskolonne 1 gelangen und die Nichtaromaten dementsprechend mit hoher Reinheit im Kopf 7 der Extraktivdestillationskolonne 7 anfallen. Diese Vorteile werden insbesondere auch bei Kohlenwasserstoffgemischen mit einem Nichtaromatengehalt unter 15% oder einem Nichtaromatengehalt unter 5% erreicht. Über der zweiten Lösungsmittelzuführung 10 in Fig. 2 ist ein weiterer Kolonnenabschnitt 11 des Kolonnenoberteils 2 angeordnet, der dazu dient, das selektive Lösungsmittel destillativ von den Nichtaromaten zu entfernen.

Im Ergebnis werden sowohl Aromaten als auch Nichtaromaten mit dem erfindungsgemäßen Verfahren in hoher Ausbeute und hoher Reinheit erhalten. Eine im Vergleich zu dem bekannten Verfahren zusätzliche Kreislauflösungsmittelmenge ist nicht erforderlich. Das Verfahren lässt sich insgesamt auch bei relativ geringem spezifischen Energieverbrauch führen.

## Patentansprüche

1. Verfahren zur Gewinnung reiner Kohlenwasserstoffe aus einem aromaten- und nichtaromatenhaltigen Kohlenwasserstoffgemisch durch Extraktivdestillation,
wobei ein aromatenreiches Kohlenwasserstoffgemisch mit einem Aromatengehalt von über 85% einer Extraktivdestillationskolonne (1) zugeführt wird,
wobei N-substituiertes Morpholin als selektives Lösungsmittel in die Extraktivdestillationskolonne (1) eingeführt wird und
wobei im Wesentlichen Nichtaromaten als Raffinat am Kopf (7) der Extraktivdestillationskolonne (1) sowie Aromaten und selektives Lösungsmittel als Extrakt aus dem Sumpf (5) der Extraktivdestillationskolonne (1) abgezogen werden,
**dadurch gekennzeichnet, dass** das selektive Lösungsmittel in zwei Teilströmen der Extraktivdestillationskolonne (1) zugeführt wird,
wobei ein erster mengenmäßig größerer Teilstrom über eine erste Lösungsmittelzuführung (4) zwischen einem Kolonnenoberteil (2) und einem Kolonnenunterteil (3) aufgegeben wird und der zweite Teilstrom mengenmäßig geregelt sowie über eine zweite Lösungsmittelzuführung (10) dem Kolonnenoberteil (2) oberhalb der ersten Lösungsmittelzuführung (4) zugeführt wird,
dass die mit dem zweiten Teilstrom zugeführte Lösungsmittelmenge 0,5 bis 10% der insgesamt zugeführten Lösungsmittelmenge beträgt und dass in einem Kolonnenabschnitt (11) des Kolonnenoberteils (2) oberhalb der zweiten Lösungsmittelzuführung (10) das selekive Lösungsmittel destillativ von den Nichtaromaten abgetrennt wird.

2. Verfahren nach Anspruch 1, **dadurch gekennzeichnet, dass** ein N-substituiertes Morpholin mit nicht mehr als 7 Kohlenstoffatomen im Substituenten als selektives Lösungsmittel verwendet wird.

3. Verfahren nach Anspruch 1 oder 2, **dadurch gekennzeichnet, dass** N-Formylmorpholin als selektives Lösungsmittel verwendet wird.

4. Verfahren nach einem der Ansprüche 1 bis 3, **dadurch gekennzeichnet, dass** das aus dem Sumpf (5) der Extraktivdestillationskolonne (1) abgezogene Extrakt aus Aromaten und selektivem Lösungsmittel einer Abtriebskolonne (6) zugeführt wird und in der Abtriebskolonne die Aromaten von dem selektiven Lösungsmittel getrennt werden.

5. Verfahren nach einem der Ansprüche 1 bis 4, **dadurch gekennzeichnet, dass** das aus dem Sumpf (5) der Extraktivdestillationskolonne (1) abgezogene selektive Lösungsmittel im Kreislauf geführt und über die Lösungsmittelzuführungen (4, 10) wieder in die Extraktivdestillationskolonne (1) eingeführt wird.

## Claims

1. A process for obtaining pure hydrocarbons by extractive distillation from a hydrocarbon mixture containing aromatics and non-aromatics,
wherein a hydrocarbon mixture which is rich in aromatics and which has an aromatic content greater than 85 % is fed to an extractive distillation column (1),
wherein N-substituted morpholine is introduced into the extractive distillation column (1) as a selective solvent, and
wherein what are substantially non-aromatics are taken off as a raffinate at the top (7) of the extractive distillation column (1), and wherein aromatics and selective solvent are taken off as an extract from the bottom (5) of the extractive distillation column (1),
**characterised in that** the selective solvent is fed to the extractive distillation column (1) in two partial streams,
wherein a first, quantitatively larger partial stream is introduced between a column upper part (2) and a column lower part (3) via a first solvent feed (4), and the second partial stream is quantitatively controlled and is fed to the column upper part (2) above the first solvent feed (4) via a second solvent feed (10),
that the amount of solvent which is fed with the second partial stream amounts to 0.5 to 10 % of the total amount of solvent fed in, and that the selective solvent is separated from the non-aromatics by distillation in a column section (11) of the column upper part (2) above the second solvent feed (10).

2. A process according to claim 1, **characterised in that** an N-substituted morpholine which contains not more than 7 carbon atoms in its substituent is used as a selective solvent.

3. A process according to claims 1 or 2, **characterised in that** N-formylmorpholine is used as a selective solvent.

4. A process according to any one of claims 1 to 3, **characterised in that** the extract, which comprises aromatics and selective solvents and which is taken off from the bottom (5) of the extractive distillation column (1), is fed to a stripping column (6) and the aromatics are separated from the selective solvents in the stripping column.

5. A process according to any one of claims 1 to 4, **characterised in that** the selective solvent which is taken of from the bottom (5) of the extractive distillation column (1) is recycled and is re-introduced into the extractive distillation column (1) via the solvent feeds (4, 10).

## Revendications

1. Procédé d'obtention d'hydrocarbures purs à partir d'un mélange d'hydrocarbures contenant des séries aromatiques et non aromatiques, par distillation extractive, dans lequel :
un mélange d'hydrocarbures riche en séries aromatiques d'une teneur en aromatiques supérieure à 85 %, est acheminé vers une colonne de distillation extractive (1),
de la morpholine N-substituée est introduite en tant que solvant sélectif dans la colonne de distillation extractive (1), et
des séries essentiellement non aromatiques sont soustraites en tant que produits raffinés à la tête de la colonne de distillation extractive ainsi que des séries aromatiques et
un solvant sélectif sont soustraits en tant qu'extrait du fond (5) de la colonne de distillation extractive (1),
**caractérisé en ce que** le solvant sélectif est acheminé en deux courants partiels vers la colonne de distillation extractive (1),
un premier courant partiel, de quantité plus grande, étant introduit, par une première arrivée de solvant (4), entre une partie supérieure de colonne (2) et une partie inférieure de colonne (3), et le deuxième courant partiel étant acheminé, en quantité réglée, et par une deuxième arrivée de solvant (10), vers la partie supérieure de colonne (2), au-dessus de la première arrivée de solvant (4),
**en ce que** la quantité de solvant, amenée au deuxième courant partiel, représente de 0,5 à 10 % de la quantité totale de solvant amené, et **en ce que** dans une portion de colonne (11) de la partie supérieure de colonne (2), au-dessus de la deuxième arrivée de solvant (10), le solvant sélectif est séparé des séries non aromatiques par distillation.

2. Procédé selon la revendication 1, **caractérisé en ce que** l'on utilise, comme solvant sélectif, une morpholine N-substituée le substituant ayant au maximum 7 atomes de carbone.

3. Procédé selon la revendication 1 ou 2, **caractérisé en ce qu'**on utilise de la N-formylmorpholine comme solvant sélectif.

4. Procédé selon l'une des revendications 1 à 3, **caractérisé en ce que** l'extrait, soustrait du fond (5) de la colonne de distillation extractive (1), constitué de séries aromatiques et d'un solvant sélectif, est acheminé vers une colonne de rectification (6), et dans celle-ci, les séries aromatiques sont séparées du solvant sélectif.

5. Procédé selon l'une des revendications 1 à 4, **caractérisé en ce que** le solvant sélectif, extrait du fond (5) de la colonne de distillation extractive (1), est recyclé et est introduit à nouveau dans la colonne de distillation extractive (1), par les arrivées de solvant (4, 10).
